(19) Europäisches Patentamt
European Patent Office
Office européen des brevets

(11) Publication number: 0 046 990 A2

# (12) EUROPEAN PATENT APPLICATION

(21) Application number: 81106684.4

(22) Date of filing: 27.08.81

(51) Int. Cl.³: C 07 D 495/04
A 61 K 31/435, C 07 D 333/36
//(C07D495/04, 333/00, 221/00)

(30) Priority: 28.08.80 JP 120109/80

(43) Date of publication of application: 10.03.82 Bulletin 82/10

(84) Designated Contracting States: BE CH DE FR GB IT LI LU NL SE

(71) Applicant: KANEBO LTD.
17-4 Sumida 5-chome Sumida-ku
Tokyo(JP)

(72) Inventor: Yamabe, Shigeru
2-7, Kamokogahara 1-chome Higashinada-ku
Hyogo-ken Kobe(JP)

(72) Inventor: Utsumi, Isamu
47, Okazaki Tenno-cho Sakyo-ku
Kyoto-fu Kyoto(JP)

(72) Inventor: Tsukamoto, Goro
2-8, Toneyama 2-chome Osaka-fu
Toyonaka(JP)

(72) Inventor: Kawashima, Tsuneo
1-57, Fukaeminami-machi 1-chome
Higashinada-ku Hyogo-ken Kobe(JP)

(72) Inventor: Uno, Toshio
1-106, 12-32, Shiginonishi 5-chome
Joto-ku Osaka-fu Osaka(JP)

(74) Representative: Kraus, Walter, Dr. et al,
Patentanwälte Dres. Kraus & Weisert Irmgardstrasse 15
D-8000 München 71(DE)

## (54) 2-Halogeno-4-alkyl-4,7-dihydro-7-oxo-thieno(3,2-b)-pyridine-6-carboxylic acids, salts thereof at the carboxyl group, pharmaceutical use thereof, and intermediates thereof.

(57) The present invention provides a 2-halogeno-4-alkyl-4,7-dihydro-7-oxo-thieno[3,2-b]pyridine-6-carboxylic acid of the general formula

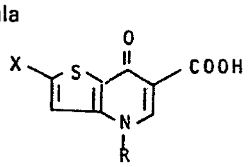

wherein X represents a halogen atom and R represents a lower alkyl group,

or a pharmaceutically acceptable salt thereof at the carboxyl group, and an antimicrobial agent comprising as an active ingredient the above compound.

The compound is useful for protecting a warm-blooded animal from bacterial infection.

The present invention also provides new intermediates for production of the compound.

EP 0 046 990 A2

Title: 2-HALOGENO-4-ALKYL-4,7-DIHYDRO-7-OXO-THIENO-(3,2-b)PYRIDINE-6-CARBOXYLIC ACIDS, SALTS THEREOF AT THE CARBOXYL GROUP, PHARMACEUTICAL USE THEREOF, AND INTERMEDIATES THEREOF

This invention relates to novel 2-halogeno-4-alkyl-4,7-dihydro-7-oxo-thieno(3,2-b)pyridine-6-carboxylic acids, pharmaceutically acceptable salts thereof at the carboxyl group, a pharmaceutical use thereof, and intermediates thereof.

Since the discovery of the excellent antimicrobial activity of Nalidixic acid of the formula

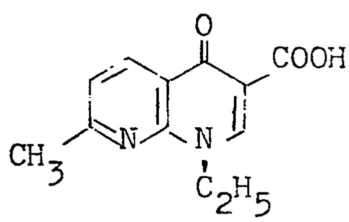

many pyridine carboxylic acids have been proposed.

U. S. Patents Nos. 3951989, 3997545 and 4079060 (west German Patent No. 2435025 corresponding to these three U. S. patents) disclose thieno(2,3-b)pyridine-carboxylic acid derivatives of the following formula

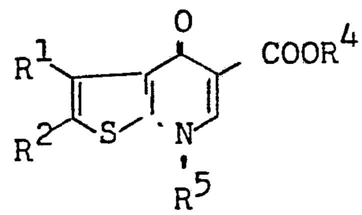

wherein $R^1$ represents hydrogen or a lower alkyl; $R^2$ represents hydrogen, a lower alkyl or a halogen; $R^1$ and $R^2$, taken together, may represent an alkylene group to form a 5- or 6-membered ring with or without alkyl substituents; each of $R^4$ and $R^5$ represents hydrogen or a lower alkyl; and their pharmaceutically acceptable salts obtainable when $R^4$ is hydrogen,

and their intermediates.

The specifications of these patents state that the thieno(2,3-b)pyridine-carboxylic acid derivatives have a broad antimicrobial spectrum against gram-positive and gram-negative bacteria. However, these specifications fail to give specific numerical values which represent their antimicrobial activity.

Antimicrobial Agents and Chemotherapy, March 1978, Vol. 13, No. 3, pages 533-535 discloses that 7-ethyl-2-methyl-4-oxo-4,7-dihydrothieno(2,3-b)pyridine-5-carboxylic acid of the following formula

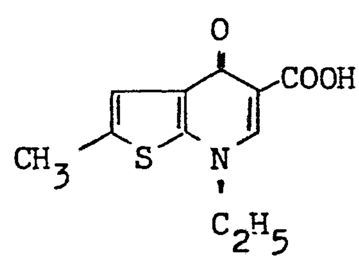

which is encompassed within the general formula disclosed in the above U. S. Patent specifications shows a similar antimicrobial activity to Nalidixic acid, and gives its specific minimum inhibitory concentrations (MIC). This literature reference, however, fails to give MIC values

of a corresponding compound in which the thieno ring is substituted by a halogen atom.

German Offenlegungsschrift No. 2447477 discloses thieno(2,3-b)pyridine carboxylic acid derivatives of the following formula

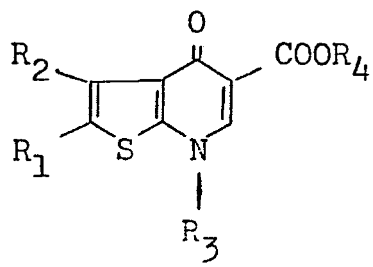

wherein $R_1$ and $R_2$ are identical or different and each represents a hydrogen atom, a nitro-group, an optionally hydroxyl-substituted aliphatic alkyl group, an O-alkyl group, an S-alkyl group, a free or substituted amino group, a halogen atom, a trifluoromethyl group or trichloromethyl group, or, taken together, represent an alkylene group having 2 to 5 carbon atoms which may contain an oxygen, sulfur or nitrogen atom (the nitrogen atom or carbon atom may be substituted), $R_3$ represents a hydrogen atom or a lower alkyl group, and $R_4$ represents a hydrogen atom, an alkyl group or an O-acylated hydroxyalkyl ester;

and salts thereof.

These compounds embrace some of the compounds disclosed in the above U. S. Patent specifications. The above German OLS discloses the antimicrobial activity of 7-ethyl-2-methyl-4-oxo-4,7-dihydrothieno(2,3-b)pyridine-5-carboxylic acid, a typical compound given therein, in comparison with that of Nalidixic acid.

J. Heterocyclic Chem. 14, 807 (1977), pages 807-812 reports a method for synthesizing thieno(2,3-b)-pyridines and thieno(3,2-b)pyridines. This article, however, fails to describe thieno(2,3-b)pyridines or (3,2-b)pyridines having a substituent on the thiophene

BAD ORIGINAL

ring, and intermediates thereof.

It is an object of this invention therefore to provide novel thieno(3,2-b)pyridine carboxylic acid derivatives, i.e. 2-halogeno-4-alkyl-4,7-dihydro-7-oxo-thieno(3,2-b)pyridine carboxylic acids, and salts thereof at the carboxyl group.

Another object of this invention is to provide a use of the above novel thieno(3,2-b)pyridine carboxylic acid derivatives as an active ingredient of an antimicrobial agent.

Still another object of this invention is to provide a series of 2-halogeno-thieno(3,2-b)pyridine carboxylic acid derivatives including compounds having better antimicrobial activity than Nalidixic acid.

Yet another object of this invention is to provide intermediates useful for the production of the novel thieno(3,2-b)pyridine carboxylic acid derivatives.

These objects and advantages of this invention are achieved by a 2-halogeno-4-alkyl-4,7-dihydro-7-oxo-thieno(3,2-b)pyridine-6-carboxylic acid of the following formula

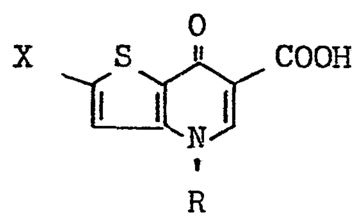

..... (I)

wherein X represents a halogen atom and R represents a lower alkyl group,

or a pharmaceutically acceptable salt thereof at the carboxyl group.

In formula (I), X is a halogen atom such as chlorine, bromine or iodine, and R is a lower alkyl group, for example an alkyl group having 1 to 3 carbon atoms such as methyl, ethyl, n-propyl or iso-propyl.

Compounds of formula (I) in which X is an iodine atom and/or R is an ethyl group are preferred.

Examples of the compounds of formula (I) are: 2-chloro-4,7-dihydro-4-ethyl-7-oxo-thieno(3,2-b)pyridine-6-carboxylic acid (X=chloro, R=ethyl), 2-bromo-4,7-dihydro-4-ethyl-7-oxo-thieno(3,2-b)pyridine-6-carboxylic acid (X=bromo, R=ethyl), 2-iodo-4,7-dihydro-4-ethyl-7-oxo-thieno(3,2-b)pyridine-6-carboxylic acid (X=iodo, R=ethyl), 2-iodo-4,7-dihydro-4-methyl-7-oxo-thieno(3,2-b)pyridine-6-carboxylic acid (X=iodo, R=methyl), and 2-iodo-4,7-dihydro-4-n-propyl-7-oxo-thieno(3,2-b)pyridine-6-carboxylic acid (X=iodo, R=n-propyl).

The pharmaceutically acceptable salts of the compounds of general formula (I) include, for example, the metal salts such as sodium, potassium and calcium salts, and the organic base salts such as ethanolamine, diethanolamine and triethylamine salts. Of these, the sodium and potassium salts are especially preferred.

All of these compounds of the invention are useful as antimicrobial agents. A compound of general formula (I) in which R is an ethyl group and X is an iodine atom is especially preferred because of its excellent antimicrobial activity.

The compounds of formula (I) can be prepared by various methods. Some examples are given below.

In accordance with Reaction Scheme A, 2-bromo-4(2,2-di(ethoxycarbonyl)ethenyl)aminothiophene (IV), an intermediate, is synthesized from 2-bromo-4-nitrothiophene (II).

Reaction Scheme A

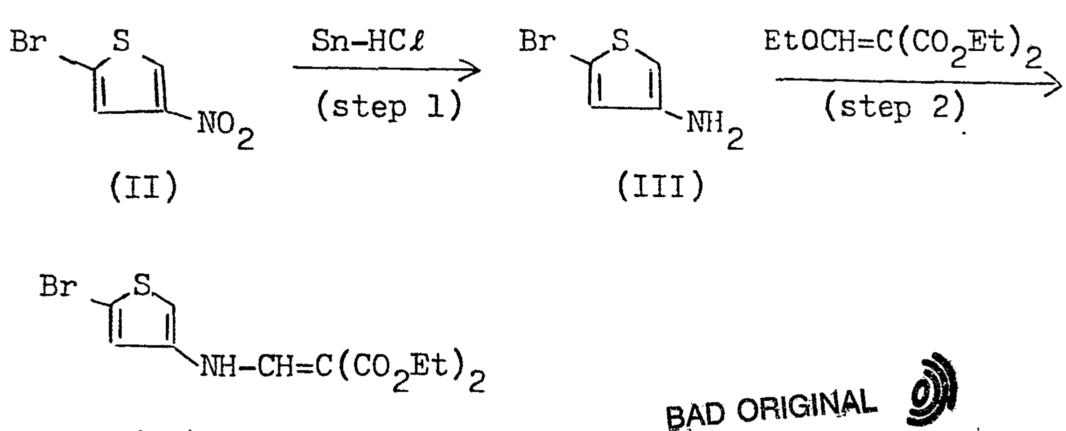


(II)

(III)

(IV)

BAD ORIGINAL

In the above scheme, Et represents an ethyl group (the same abbreviation will be used hereinbelow).

First, 2-bromo-4-nitrothiophene (II) synthesized by a known method (Journal of the Chemical Society of Japan, Vol. 78, pages 788-792, 1957) is used as a starting material and reduced to 4-amino-2-bromothiophene (III) in accordance with step 1. Then, according to step 2, the compound (III) is reacted with diethyl ethoxymethylenemalonate to give 2-bromo-4-(2,2-di(ethoxycarbonyl) ethenyl)aminothiophene (IV).

The reducing reaction of 2-bromo-4-nitrothiophene (II) (step 1) is conveniently carried out by using tin/hydrochloric acid (Sn-HCl). After the reaction, the reaction product is dissolved in water and washed with an organic solvent such as diethyl ether in order to remove the by-product 4-amino-2-bromo-5-chlorothiophene. The aqueous layer was then made basic and extracted with an organic solvent to isolate the 4-amino-4-bromothiophene (III).

Preferably, immediately after preparation, the 4-amino-2-bromothiophene (III) is submitted to the reaction with diethyl ethoxymethylenemalonate (step 2) because it is very susceptible to decomposition. In order to avoid decomposition of the compound (III) during the reaction, the reaction is carried out preferably in a stream of nitrogen. The reaction is performed in an organic solvent such as toluene, xylene, chloroform, dichloroethane or dimethyl formamide, or in the absence of a solvent. The reaction temperature is usually in the range of 65 to 150$^{o}$C, preferably 110 to 120$^{o}$C.

As a result, a novel compound, a 2-bromo-4(2,2-di(alkoxycarbonyl)-ethenyl)aminothiophene (IV), is obtained in high yields. The compound (IV) is useful as an intermediate for the production of various compounds as well as the compound (I) of the invention.

The desired compound of formula (I) is then

BAD ORIGINAL

produced by using the intermediate compound (IV).

The process is described with reference to Reaction Scheme B taking up as an example the production of a compound of formula (I) in which X is a chlorine atom (formula (I-a)).

Reaction Scheme B

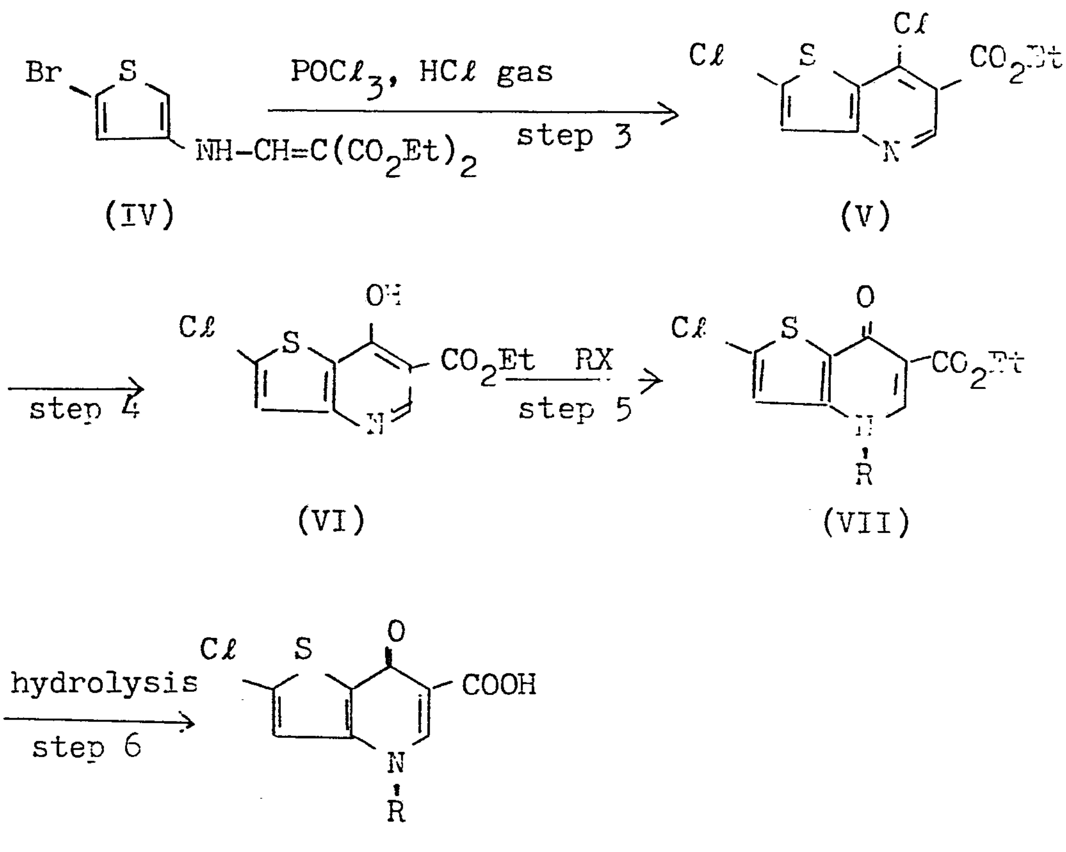


(IV) (V)

(VI) (VII)

(I-a)

wherein R is a lower alkyl group and X is a halogen atom.

The reaction of step 3 for the production of the compound (V) from the compound (IV) is carried out by heating the compound (IV) with an excessive amount (preferably 10 to 20 moles per mole of the compound of formula (IV)) of phosphorus oxychloride at a temperature of preferably 105 to 110°C for several hours, for example 2 to 5 hours, and then heating it further for 1 to 7 days, preferably 2 to 3 days, while occasionally introducing hydrogen chloride gas. Thus, a novel compound (V) having

a chlorine atom both at the 2- and 7-positions is obtained.

A 2-chloro-7-hydroxy derivative (VI) thereof can be obtained by heating the compound of formula (V) at a temperature between 50°C and 160°C in sodium acetate-acetic acid, sodium acetate-dimethyl sulfoxide, hydrochloric acid-ethanol, etc. in accordance with step 4. The use of sodium acetate-acetic acid is most preferred, and in this case, it is preferred to use 1 to 5 moles of sodium acetate per mole of the compound of formula (V) and 10 to 40 parts by weight of acetic acid per part by weight of the compound of formula (V). Heating for 5 to 10 hours gives the 2-chloro-7-hydroxy derivative (VI) in high yields. The compound (VI) is a novel compound.

Action of a lower alkyl halide of the formula RX where R and X are as defined on the 2-chloro-7-hydroxy derivative (VI) in accordance with the alkylation of step 5 gives a 2-chloro-4,7-dihydro-4-alkyl-7-oxo-thieno(3,2-b)-pyridine-6-carboxylic acid ethyl ester (VII). Then, the novel compound (VII) is hydrolyzed in a usual manner in accordance with step 6 to give the desired compound of formula (I-a) in which X is a chlorine atom.

Alkylation (step 5) of the compound (VI) can be performed in a conventional manner. For example, 1 mole of the compound (VI) is reacted with 1 to 3 moles of the alkyl halide in the presence of a base in an inert solvent such as dimethyl formamide or dimethyl sulfoxide at room temperature to 150°C.

Examples of the base used in step 5 are alkali metal salts or hydroxides such as potassium carbonate, potassium hydroxide and sodium hydroxide, sodium ethoxide, potassium butoxide and sodium hydride.

Instead of the lower alkyl halide, a dialkyl sulfate or lower alkyl tosylate having a corresponding lower alkyl group may be used in the alkylation of step 5.

The resulting 2-chloro-4,7-dihydro-4-alkyl-7-oxo-thieno(3,2-b)pyridine-6-carboxylic acid ethyl ester (VII)

BAD ORIGINAL

is then treated in accordance with step 6 in 1N sodium hydroxide, for example, at a temperature of 50 to 100°C and for 5 to 60 minutes.

The production of the compounds (I-b) or (I-c) of the invention corresponding to formula (I) in which X is a bromine or iodine atom is described with reference to Reaction Scheme C.

Reaction Scheme C

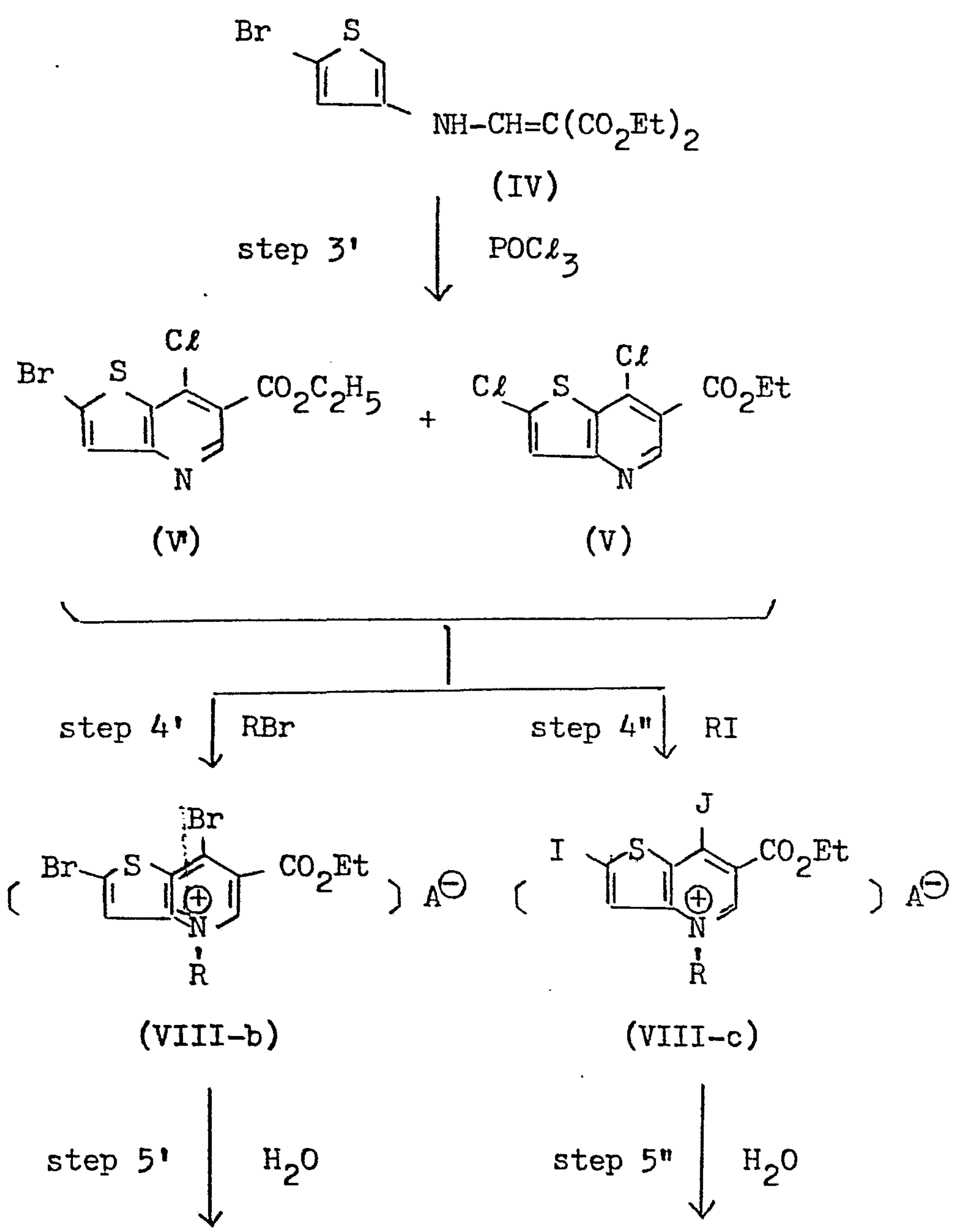

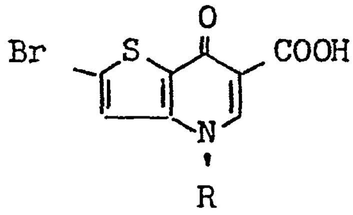

(I-b)

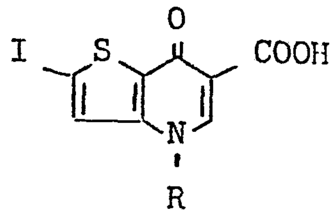

(I-c)

In the above scheme, R is as defined, and $A^{\ominus}$ represents a counter halogen anion.

When the compound (IV) is heated with an excessive amount (preferably 5 to 10 moles per mole of the compound (IV)) at a bath temperature of 80 to 140°C, preferably about 110°C, for 1 to 5 hours, a cyclized product (V) in which the 2-position is chlorine atom and a cyclized product (V') in which the 2-position is bromine atom are obtained.

The reaction of step 3' is the same as step 3 of Reaction Scheme B in that the compound (IV) is reacted with phosphorus oxychloride. These reactions, however, differ from each other in that the reaction conditions in step 3' are milder than those in step 3, and step 3 uses $HC\ell$ gas whereas step 3' does not.

According to Reaction Scheme C, a mixture of the compounds (V) and (V') is then reacted in a usual manner with a lower alkyl bromide (RBr) such as ethyl bromide and a lower alkyl iodide (RI) such as ethyl iodide in accordance with steps 4' and 4" to give a quaternary ammonium salt (VIII-b) in which both of the 2- and 7-positions are bromine atoms, and a quaternary ammonium salt (VIII-c) in which both the 2- and 7-positions are iodine atom in good yields. The compounds (VIII-b) and (VIII-c) are also novel.

The lower alkyl bromide is used normally in an amount of 4 to 40 moles per mole of the compound (V+V'), and the reaction is preferably carried out in an aprotic organic solvent such as dimethyl formamide or dimethyl

sulfoxide.

The lower alkyl iodide is used in a large excess with respect to the compound (V+V'). In other words, the reaction is usually carried out by using the lower alkyl iodide both as a reagent and a solvent.

The compounds (VIII-b) and (VIII-c) obtained in steps 4' and 4" are then respectively hydrolyzed in a usual manner by steps 5' and 5" to give the desired compounds (I-b) and (I-c) of the invention. The hydrolysis in steps 5' and 5" can be carried out under similar conditions to those in step 6.

The 2-halogeno-4-alkyl-4,7-dihydro-7-oxo-thieno[3,2-b]pyridine-6-carboxylic acids of formula (I) including formulae (I-a), (I-b) and (I-c) may, if required, be converted to their salts at the carboxyl group in a manner known per se.

These compounds provided by the process of this invention have a broad antimicrobial spectrum, and those of formula (I) in which X is an iodine atom exhibit better antimicrobial activity than Nalidixic acid.

The antimicrobial activities (MIC) of a compound of general formula (I) in which X is a chlorine atom and R is an ethyl group (compound A), a compound of general formula (I) in which X is a bromine atom and R is an ethyl group (compound B) and a compound of formula (I) in which X is an iodine atom and R is an ethyl group (compound C) are determined by the antimicrobial test described in Chemotherapy, Tokyo, Vol. 23, No. 8, pages 1 to 2 (1975) and are shown in the following Table 1. Nalidixic acid is used as a control.

Table 1

| Assay microorganism | MIC (μg/ml) | | | |
|---|---|---|---|---|
| | Compound A | Compound B | Compound C | Nalidixic acid |
| Staphylococcus aureus 209-P | >100 | >100 | 25 | >100 |
| Bacillus subtillis ATCC 6633 | 12.5 | 6.25 | 1.56 | 3.13 |
| Escherichia coli NIHJ | 1.56 | 1.56 | 1.56 | 1.56 |
| Escherichia coli K-12 | 12.5 | 6.25 | 3.13 | 6.25 |
| Klebsiella pneumoniae IFO 3512 | 6.25 | 3.13 | 1.56 | 1.56 |
| Serratia marcescens IFO 3046 | 3.13 | 1.56 | 0.78 | 1.56 |
| Proteus vulgaris OX-19 | 12.5 | 6.25 | 0.78 | 3.13 |
| Proteus mirabilis 1287 | 6.25 | 3.13 | 0.78 | 3.13 |

The compounds of this invention have low toxicity. For example, the $LD_{50}$ of the compound C is 2,800 mg/kg (in oral administration to mice).

Accordingly, the present invention also provides an antimicrobial agent comprising as an active ingredient the 2-halogeno-4-alkyl-4,7-dihydro-7-oxo-thieno[3,2-b]-pyridine-6-carboxylic acid of formula (I) or a pharmaceutically acceptable salt thereof at the carboxyl group.

The antimicrobial agent of the invention is administered (preferably orally) to warm-blooded animals, such as humans, with various bacterial infections either as such or together with a pharmaceutically acceptable

BAD ORIGINAL

carrier or diluent such as a vehicle, excipient, binder or lubricant.

The antimicrobial agent is administered in a dose of about 5 to about 180 mg/kg.day, preferably about 10 to about 100 mg/kg.day as an active ingredient. Depending upon the condition of a subject to be treated, the dose may be administered at a time or in several portions per day, for example 2 to 4 times a day.

The antimicrobial agent of the invention can be advantageously administered in the form of fine granules, granules, tablets, capsules, etc.

The following Examples illustrate the present invention in more detail.

Example 1

Production of 4-amino-2-bromothiophene (III):-

Conc. hydrochloric acid (70 ml) was added to 6.5 g of 2-bromo-4-nitrothiophene (II), and the mixture was vigorously stirred in an ice water bath. Then, 6.5 g of powdery tin was added in portions so that the reaction temperature reached not more than 10°C. After the addition, the mixture was stirred further for 15 minutes, and the resulting creamy powder (7 g) was collected by filtration. Water (80 ml) and 80 ml of ether were added, and the mixture was shaken well to separate it into an ethereal layer and an aqueous layer. The ethereal layer was shaken with a small amount of water, and combined with the aforesaid aqueous layer, and washed with a small amount of ether. To the resulting aqueous layer was added 200 ml of ether, and the mixture was cooled with ice water in an atmosphere of nitrogen. With stirring, 30 ml of a 33% aqueous solution of sodium hydroxide was gradually added dropwise. The ethereal layer was collected, washed with water, and dried over anhydrous magnesium sulfate. Then, a greater portion of the ether was distilled off to give 3 g of 4-amino-2-bromothiophene (oily substance) as an ether solution. Since the 4-amino-2-bromothiophene (III) is susceptible to decomposition, it was immediately

BAD ORIGINAL

used in the subsequent condensation reaction as an ether solution.

Example 2

Production of 2-bromo-4-[2,2-di(ethoxycarbonyl)ethenyl]aminothiophene (IV):-

2,6 g of 4-amino-2-bromothiophene (III) was dissolved in 10 ml of toluene, and 3.2 g of diethyl ethoxymethylenemalonate was added. The mixture was heated at a bath temperature of 110 to 120°C for 15 minutes in a stream of nitrogen. Then, the solvent was distilled off to give 5 g of a brown solid. Recrystallization from hexane afforded 3.6 g (yield 70%) of 2-bromo-4-[2,2-di(ethoxycarbonyl)ethenyl]aminothiophene (IV) as light brown needles.

Melting point: 81 - 82°C

IR (KBr), $\nu$max($cm^{-1}$): 3170, 1685, 1640, 1605.

NMR ($CDCl_3$), $\delta$(ppm): 1.3(6H, m), 4.2(4H, m), 6.6 (1H, d), 6.8 (1H, d), 8.1 (1H, d), 11.0 (1H, d).

Example 3

Production of 2-chloro-4,7-dihydro-4-ethyl-7-oxo-thieno[3,2-b]pyridine-6-carboxylic acid (compound A):-

(1) Production of ethyl-2,7-dichloro-thieno[3,2-b]pyridine-6-carboxylate (V):-

Phosphorus oxychloride (20 ml) was added to 2.0 g of 2-bromo-4-[2,2-di(ethoxycarbonyl)ethenyl]aminothiophene (IV) and the mixture was heated at a bath temperature of 110°C for 3.5 hours. The reaction mixture was then allowed to cool. Hydrogen chloride gas was introduced for 30 minutes into the reaction mixture, and then the bath temperature was maintained at 105 to 110°C. With occasional blowing of hydrogen chloride gas, the mixture was heated for 50 hours. The reaction mixture was then concentrated, and ice water was added to the residue. The precipitate in the form of a brown powder was collected by filtration, washed with water and dried to give 1.6 g of crude ethyl 2,7-dichloro-thieno[3,2-b]-

pyridine-6-carboxylate (V). This product was treated with activated carbon in benzene. The benzene was distilled off, and the resulting residue was recrystallized from hexane to give 1.0 g of pale yellow needles.

Melting point: 77.5 - 80°C
IR(KBr), νmax($cm^{-1}$): 1725.
NMR($CDCl_3$), δ(ppm): 1.45 (3H, s) 4.40 (2H, q), 7.35 (1H, s), 9.0 (1H, s).

(2) Production of ethyl-2-chloro-7-hydroxy-thieno-(3,2-b)pyridine-6-carboxylate (VI):-

Five grams of the ethyl 2,7-dichloro-thieno-(3,2-b)pyridine-6-carboxylate (V) and 7.5 g of sodium acetate trihydrate were added to 150 ml of acetic acid, and the mixture was refluxed for 8 hours. After the reaction, acetic acid was distilled off, and water was added. The resulting pale brown crystals were collected by filtration, washed with water and dried. Recrystallization from dimethyl formamide gave 3.9 g (yield 83%) of ethyl 2-chloro-7-hydroxythieno(3,2-b)pyridine-6-carboxylate (VI) as colorless needles.

Melting point: 272- 273°C
IR (KBr), νmax ($cm^{-1}$): 1700, 1610.
NMR ($CF_3COOD$), δ(ppm): 1.6 (3H, t), 4.75 (2H, q), 7.7 (1H, s), 9.2 (1H, s).

(3) Production of ethyl 2-chloro-4,7-dihydro-4-ethyl-7-oxo-thieno(3,2-b)pyridine-6-carboxylate (VII):-

Dimethyl formamide (120 ml) was added to 3 g of ethyl 2-chloro-7-hydroxy-thieno(3,2-b)pyridine-6-carboxylate (VI) and 4.8 g of potassium carbonate, and the mixture was heated at a bath temperature of 60°C with stirring. Then, 3.6 g of ethyl iodide was gradually added dropwise, and after the addition, the reaction was carried out for 3 hours. Then, the excess of ethyl iodide and the solvent were removed, and 150 ml of water and 200 ml of ethyl acetate were added. The mixture was well shaken. The organic layer was separated, washed with water and dried over anhydrous magnesium sulfate.

BAD ORIGINAL

The solvent was distilled off to give 2.9 g of colorless crystals. Recrystallization from ethyl acetate gave 2.1 g (yield 63%) of ethyl 2-chloro-4,7-dihydro-4-ethyl-7-oxo-thieno(3,2-b)pyridine-6-carboxylate (VII) as colorless needles.

Melting point: 204 - 206°C

IR (KBr), ν max ($cm^{-1}$): 1725, 1610.

NMR ($CDCl_3$), δ (ppm): 1.5 (6H, q), 4.2 (4H, sextet) 7.4 (1H, s), 8.1 (1H, s).

(4) Production of 2-chloro-4,7-dihydro-4-ethyl-7-oxo-thieno(3,2-b)pyridine-6-carboxylic acid (compound A):-

60 ml of a 1N aqueous solution of sodium hydroxide was added to 1.5 g of ethyl 2-chloro-4,7-dihydro-4-ethyl-7-oxo-thieno(3,2-b)pyridine-6-carboxylate (VII), and the mixture was heated at a bath temperature of 90 to 95°C for 10 minutes with stirring. The reaction mixture was cooled with ice water, and acidified with 3N hydrochloric acid. The resulting colorless crystals were collected by filtration, washed with water and dried to give 1.34 g of crude 2-chloro-4,7-dihydro-4-ethyl-7-oxo-thieno(3,2-b)-pyridine-6-carboxylic acid (compound A). Recrystallization from dimethyl formamide/ethanol gave 1.2 g (yield 89%) of colorless needles.

Melting point: 290.5 - 293°C

IR (KBr), νmax ($cm^{-1}$): 3440, 1720, 1610.

NMR (DMSO-$d_6$), δ(ppm): 1.4 (3H, t), 4.5 (2H, q), 8.0 (1H, s), 8.9 (1H, s).

Example 4

Production of 2-bromo-4,7-dihydro-4-ethyl-7-oxo-thieno(3,2-b)pyridine-6-carboxylic acid:-

(1) Production of a mixture of ethyl 2,7-dichloro-thieno(3,2-b)pyridine-6-carboxylate (V) and ethyl 2-bromo-7-chloro-thieno(3,2-b)pyridine-6-carboxylate (V'):-

Phosphorus oxychloride (90 ml) was added to 9.0 g of 2-bromo-4-(2,2-di(ethoxycarbonyl)ethenyl)amino-thiophene (IV), and the mixture was heated at a bath temperature of 110°C for 4 hours. The reaction mixture

BAD ORIGINAL

was then concentrated, and poured into about 600 ml of ice water, followed by neutralization with a saturated aqueous solution of sodium hydrogen carbonate. The resulting precipitate in the form of a pale brown powder was collected by filtration, washed with water and dried to give 7.2 g of a mixture of crude compounds (V) and (V'). Recrystallization from hexane gave 6 g of a mixture of purified compounds (V) and (V') as pale yellow crystals.

Melting point: 84.5 - 85.5°C

IR (KBr), $\nu$max ($cm^{-1}$): 1725.

The measurement of NMR of this mixture showed that the ratio of the chloro-product (V) to the bromo-product (V') in the crystalline mixture was about 2:3.

NMR ($CDCl_3$), $\delta$(ppm): 1.4 (3H, t), 4.4 (2H, q), 7.35 (0.4H, s), 7.5 (0.6H, s), 9.0 (1H, s).

(2) Production of 2-bromo-4,7-dihydro-4-ethyl-7-oxo-thieno(3,2-b)pyridine-6-carboxylic acid (compound B):-

1.0 g of the mixture of compounds (V) and (V') obtained in (1) above was dissolved in 30 ml of dimethyl formamide, and 4.8 ml of ethyl bromide was added. The mixture was heated at 130°C for 24 hours in a sealed tube.

The solvent was distilled off and the resulting orange residue was washed with ethyl acetate to give 1.17 g of the corresponding pyridinium salt. To the product was added 10 ml of a 1N aqueous solution of sodium hydroxide, and the mixture was heated at 95 to 97°C for 15 minutes. The reaction mixture was cooled to room temperature, and the resulting insoluble precipitate was separated by filtration.

The filtrate was acidified with 4N hydrochloric acid. The resulting precipitate was collected by filtration, and washed with water and then with a small amount of ethanol to give 711 mg of a crude product.

Recrystallization from ethanol gave 60 mg of 2-bromo-4,7-dihydro-4-ethyl-7-oxo-thieno(3,2-b)pyridine-6-carboxylic acid (compound B) as colorless needles.

BAD ORIGINAL

Melting point: 292 - 293.5°C
IR (KBr), νmax ($cm^{-1}$): 3080, 1715, 1605.
NMR (DMSO-$d_6$), δ(ppm): 1.41 (3H, t), 4.50 (2H, q), 8.05 (1H, s), 8.83 (1H, s).

Example 5

Production of 2-iodo-4,7-dihydro-4-ethyl-7-oxo-thieno(3,2-b)pyridine-6-carboxylic acid (compound C):-

4.7 g of a mixture of compounds (V) and (V') obtained as in Example 4, (1) was dissolved in 70 ml of ethyl iodide, and the mixture was refluxed for 25 hours. After cooling, 3 g of 2,7-diiodo-thieno(3,2-b)pyridinium salt (VIII-c) as pale yellow precipitate was collected by filtration. The filtrate was further refluxed for 25 hours, and allowed to cool to give 2 g of 2,7-diiodo-thieno(3,2-b)pyridinium salt further.

Decomposition point: 215 - 216.5°C
NMR (DMSO-$d_6$), (ppm): 1.1 - 1.7 (6H, m), 3.9 - 4.5 (4H, m), 7.7 (1H, s), 8.3 (1H, s).

100 ml of 1N sodium hydroxide was added to 5 g of the resulting yellow precipitate (VIII-c), and the mixture was stirred. The mixture was heated at a bath temperature of 87 to 92°C for 15 minutes, and then allowed to cool. Then, 3N hydrochloric acid was added to the reaction mixture to make it acidic, and the resulting precipitate in powder form was collected by filtration. The precipitate was washed with water and a small amount of ethanol successively, and dried to give 2.9 g of 2-iodo-4,7-dihydro-4-ethyl-7-oxo-thieno-(3,2-b)pyridine-6-carboxylic acid (compound C) as a pale green powder. Recrystallization from dimethyl formamide/water gave 2.4 g of colorless fine needles.

Melting point: 251 - 254°C
IR (KBr), νmax ($cm^{-1}$): 3440, 1720, 1610.
NMR (DMSO-$d_6$), δ(ppm): 1.4 (3H, t), 4.4 (2H, q), 7.9 (1H, s), 8.6 (1H, s).

Example 6

BAD ORIGINAL

Preparation of capsules:

| | |
|---|---|
| Compound C | 250 g |
| Corn starch | 60 g |
| Lactose | 35 g |
| Magnesium stearate | 5 g |
| | 350 g |

The above components were blended and granulated and filled into 1,000 capsules by a conventional method.

Example 7

Preparation of tablets:-

| | |
|---|---|
| Compound C | 250 g |
| Corn starch | 46 g |
| Microcrystalline cellulose | 100 g |
| Magnesium stearate | 4 g |
| | 400 g |

The above components were blended, granulated and tableted by conventional methods. One thousand tablets each weighing 400 mg were formed.

BAD ORIGINAL

WHAT WE CLAIM IS:

1. A 2-halogeno-4-alkyl-4,7-dihydro-7-oxo-thieno(3,2-b)pyridine-6-carboxylic acid of the general formula

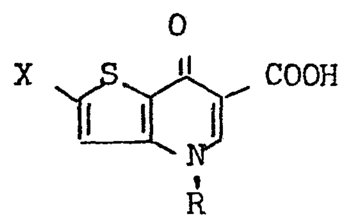

(I)

wherein X represents a halogen atom and R represents a lower alkyl group,

or a pharmaceutically acceptable salt thereof at the carboxyl group.

2. The compound of claim 1 wherein R in formula (I) is an ethyl group.

3. The compound of claim 1 or 2 wherein X in formula (I) is an iodine atom.

4. An antimicrobial agent comprising as an active ingredient a 2-halogeno-4-alkyl-4,7-dihydro-7-oxo-thieno(3,2-b)pyridine-6-carboxylic acid of the general formula

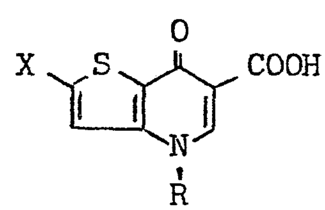

(I)

wherein X represents a halogen atom and R represents a lower alkyl group,

or a pharmaceutically acceptable salt thereof at the carboxyl group.

5. The antimicrobial agent of claim 4 wherein R in formula (I) is an ethyl group.

6. The antimicrobial agent of claim 4 or 5 wherein X in formula (I) is an iodine atom.

7. A method for protecting a warm-blooded animal from bacterial infection, which comprises administering

BAD ORIGINAL

to the warm-blooded animal a 2-halogeno-4-alkyl-4,7-dihydro-7-oxo-thieno(3,2-b)pyridine-6-carboxylic acid of the general formula

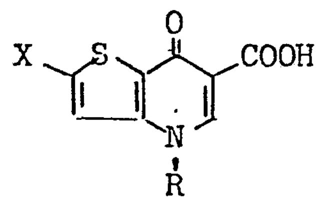

(I)

wherein X represents a halogen atom, and R represents a lower alkyl group,

or a pharmaceutically acceptable salt thereof at the carboxyl group.

8. 2,7-Dichloro-thieno(3,2-b)pyridine-6-carboxylic acid ethyl ester of the formula

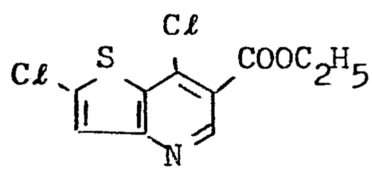

(V).

9. 2-Chloro-7-hydroxy-thieno(3,2-b)pyridine-6-carboxylic acid ethyl ester of the formula

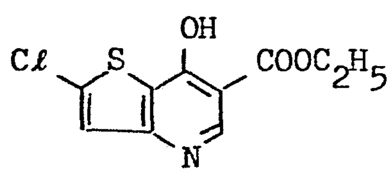

(VI).

10. 2-Bromo-4-(2,2-di(ethoxycarbonyl)ethenyl)amino-thiophene of the following formula

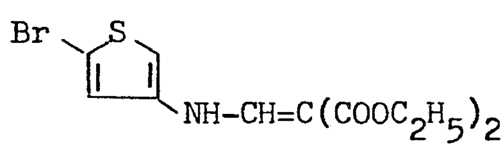

(IV).

BAD ORIGINAL